# EUROPEAN PATENT APPLICATION

(11) **EP 3 868 368 A1**
(43) Date of publication of application: **25.08.2021**
(21) Application number: 20158749.0
(22) Date of filing: 21.02.2020
(51) Int. Cl.: A61K 31/18, A61K 31/00, A61K 31/573, A61K 45/06, A61P 11/06

(54) **CXCL8 (INTERLEUKIN-8) ACTIVITY INHIBITOR AND CORTICOSTEROID COMBINATION AND PHARMACEUTICAL COMPOSITION AND USE THEREOF**

(71) Applicant: Dompe' Farmaceutici S.P.A., 20122 Milan (IT)
(72) Inventor: RUSSO, Remo de Castro, 31270-901 Belo Horizonte - Minas Gerais (BR); TEIXEIRA, Mauro Martin, 31270-901 Belo Horizonte - Minas Gerais (BR); BRANDOLINI, Laura, 67100 L'Aquila (IT); ALLEGRETTI, Marcello, 67100 L'Aquila (IT)
(74) Representative: Mauri, Elisabetta Maria Ester

(57) **Abstract**

The present invention relates to a combination of a CXCL8 activity inhibitor and a corticosteroid, and a pharmaceutical composition thereof, for use in the treatment of corticosteroid-insensitive asthma as well as to a method for treating corticosteroid-insensitive asthma by administration of said pharmaceutical composition.

## Description

### FIELD OF THE INVENTION

The present invention relates to a synergistic combination of a CXCL8 (Interleukin-8) activity inhibitor and a corticosteroid and its use in therapy. More in particular, the present invention relates to the use of a synergistic combination of a CXCL8 activity inhibitor, such as ladarixin, and a corticosteroid, such as dexamethasone, for the treatment of corticosteroid-resistant asthma.

### STATE OF THE ART

Asthma is a chronic inflammatory allergic airway disorder that causes variable airflow obstruction in association with airway hyperreactivity (AHR) and mucus overproduction (Fanta CH. Asthma. N Engl J Med 2009; 360:1002-14). Asthma affects up to 300 million people worldwide, and the prevalence of asthma continues to rise especially in Africa, Latin America, Eastern Europe and Asia countries.

Most asthmatic patients have a mild-to-moderate disease that can be well controlled with standard treatments, including the regular use of inhaled corticosteroids (ICSs), often combined with short-acting inhaled β2-agonists (SABAs) or long-acting inhaled β2-agonists (LABAs).

However, a subset of patients with asthma experiences a complete absence of or a very limited response to traditional corticosteroid therapy. This type of asthma is referred to as corticosteroid-insensitive asthma or corticosteroid-resistant asthma. In these patients, high doses of corticosteroids are frequently prescribed without pharmacological evidence for their utility and with increasing side effects. In many of these patients asthma remains uncontrolled and is associated to a particularly high risk of exacerbations, hospitalization and death, and a severly impaired quality of life (Chanez P et al, J Allergy Clin Immunol 2007; 119:1337-48; Dockrell M et al, Allergy 2007; 62:134-41).

Numerous efforts have been made in order to identify effective treatments for this group of asthma patients.

For example, a clinical trial conducted with anti-IL-5 antibodies (mepolizumab) showed a reduction in exacerbation frequency and improved patients quality of life according to the AQLQ (Asthma Quality of Life Questionnaire). In addition to the action on asthma, this trial also showed a therapeutic effect against polypous rhinosinusopathy (Haldar P et al., Mepolizumab and exacerbations of refractory eosinophilic asthma (N Engl J Med. 2009 Mar 5;360(10):973-84).

Currently, the anti-IgE agent, omalizumab, the anti-IL-5 agents mepolizumab and reslizumab, and the anti-IL-5α receptor, benralizumab are the biologic drugs approved as add-on therapy for severe asthma with an eosinophilic phenotype (Ther Adv Respir Dis 2018, Vol. 12: 1-12DOI: 10.1177/1753466618808490).

US10420792 discloses a method for treating corticosteroid-insensitive asthma in a patient displaying a Th1 immune profile, which comprises reducing Interferon Regulatory Factor 5 (IRF5) activity, for example using antisense or RNA interference reagents (such as miRNA or siRNA) or an antibody or antibody fragment.

US9078894 discloses a method for the treatment of severe persistent corticosteroid-resistant asthma by administration of a tyrosine kinase inhibitor or a mast cell inhibitor, such as masitinib or a salt thereof.

In spite of the research efforts conducted up to now, an urgent need still exists for a new and more effective medical treatment that allows adequate control of corticosteroid-resistant asthma.

Chemokines are a family of structurally related peptides that regulate inflammation through cell-surface G protein-coupled receptors on the surface of leukocytes. They mediate diverse biological and biochemical activities, for example endothelial adhesion, directed migration, and activation of cytotoxic activities such as the respiratory burst and exocytosis (Baggiolini, M. et al, (1997) Annu. Rev. Immunol. 15, 675-705)

Chemokines have been classified into four families (C, CC, CXC, and CX3C) based on the number and positions of the N-terminally conserved cysteine residues. Most chemokines bind and activiate more than one chemokine receptor, and many chemokine receptors are activated by multiple chemokines (Murphy, P. M. et al (2000) Pharmacol. Rev. 52, 145-176).

Interleukin-8 (IL-8; CXCL8) is considered a major mediator of PMN (Polymorphonuclear Neutrophils) recruitment and is involved in several pathologies including psoriasis, rheumatoid arthritis, chronic obstructive pulmonary disease and reperfusion injury in transplanted organ.

The biological activity of CXCL8 is mediated by the interaction with two receptors, CXCR1 and CXCR2, that are expressed on the surface of human PMNs. CXCR1 is selective for CXCL8, whereas CXCR2 is a more promiscuous receptor, binding a number of different cytokines and chemokines such as CXCL1, CXCL2, CXCL3, CXCL5, CXCL6, and CXCL7 (Baggiolini, M., (2000) Immunol. Rev. 177, 5-7).

### SUMMARY OF THE INVENTION

The Applicant has now surprisingly found that in a corticosteroid-resistant asthma animal model, the association of a CXCL8 activity inhibitor, such as ladarixin, to the traditional treatment with a corticosteroid, such as dexamethasone, is able to restore the anti-inflammatory effect of dexamethasone via a synergistic mechanism, preventing neutrophilic airway inflammation and protein leakage, tissue remodeling, bronchial hyperreactivity and improved pulmonary mechanics.

Then, in a first aspect, the present invention relates to a combination of a CXCL8 activity inhibitor and a corticosteroid and to said combination for use in the treatment of corticosteroid-insensitive asthma.

In a second aspect, the present invention relates to a pharmaceutical composition comprising a combination according to the first object of the invention and at least one inert pharmaceutically acceptable excipient, for use in the treatment of corticosteroid-insensitive asthma.

In a third aspect, the present invention relates to a method of treating corticosteroid-insensitive asthma in a subject in need thereof, comprising administering to the subject a pharmaceutical composition comprising a combination of a CXCL8 activity inhibitor and a corticosteroid, and at least one inert pharmaceutically acceptable excipient.

### BRIEF DESCRIPTION OF THE FIGURES

The embodiments of the present invention will now be described, by way of example, with reference to the accompanying drawings, in which:

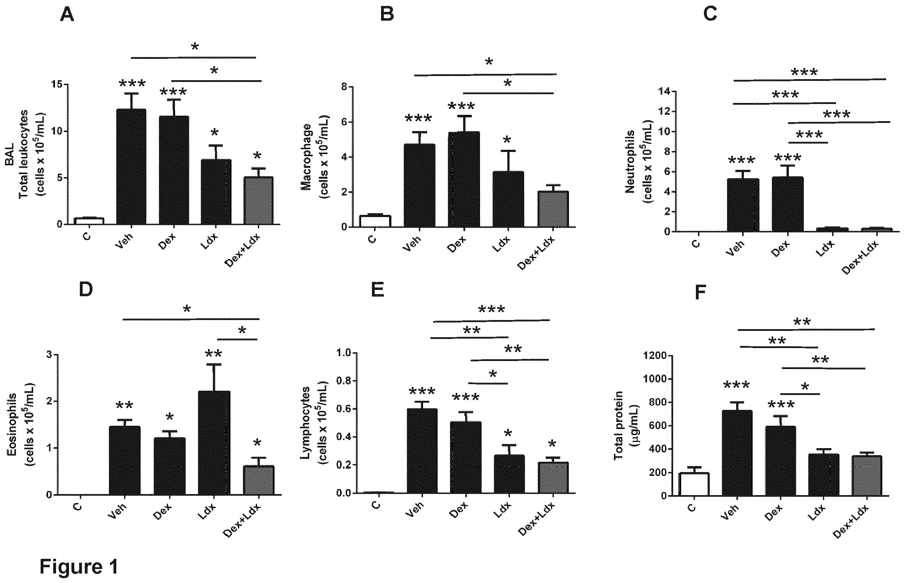

**Figure 1** shows the data obtained by the assessment of blood leakage into airways of: total leukocytes (panel A), macrophages (panel B), neutrophils (panel C), eosinophils (panel D), lymphocytes (panel E) and total protein (panel F), measured as described in Example 1 a), in a mouse model of corticosteroid-resistant asthma, after treatment with vehicle (Veh), dexamethasone (Dex), ladarixin (Ldx), or dexamethasone and ladarixin (Dex+Ldx);

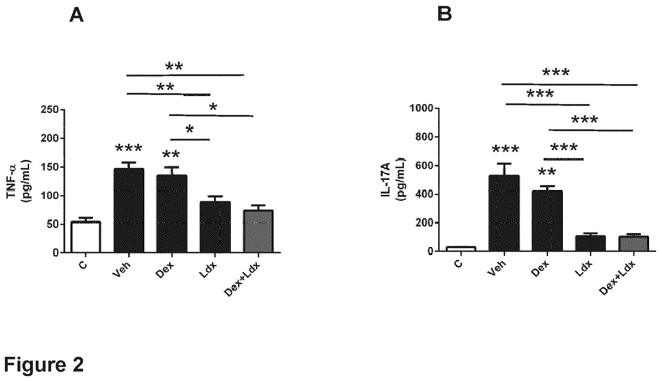

**Figure 2** shows TNF-α (panel A) and IL-17A (panel B) levels in lung parenchyma, measured as described in Example 1b) in a mouse model of corticosteroid-resistant asthma, after treatment with vehicle (Veh), dexamethasone (Dex), ladarixin (Ldx), or dexamethasone and ladarixin (Dex+Ldx);

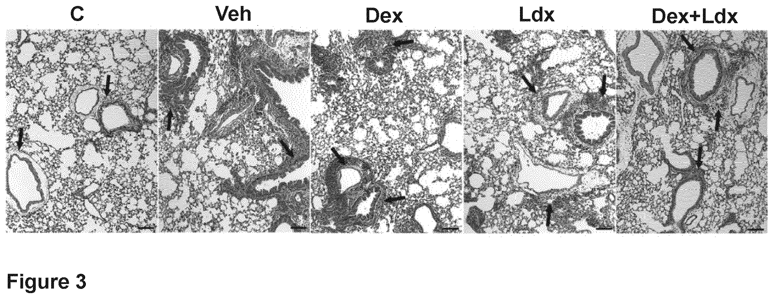

**Figure 3** shows histological sections of lung (H&E dye) obtained as described in Example 1d-400x magnification) from a mouse model of corticosteroid-resistant asthma, after treatment with vehicle (Veh), dexamethasone (Dex), ladarixin (Ldx), or dexamethasone and ladarixin (Dex+Ldx), wherein arrows indicate peribronchial inflammation;

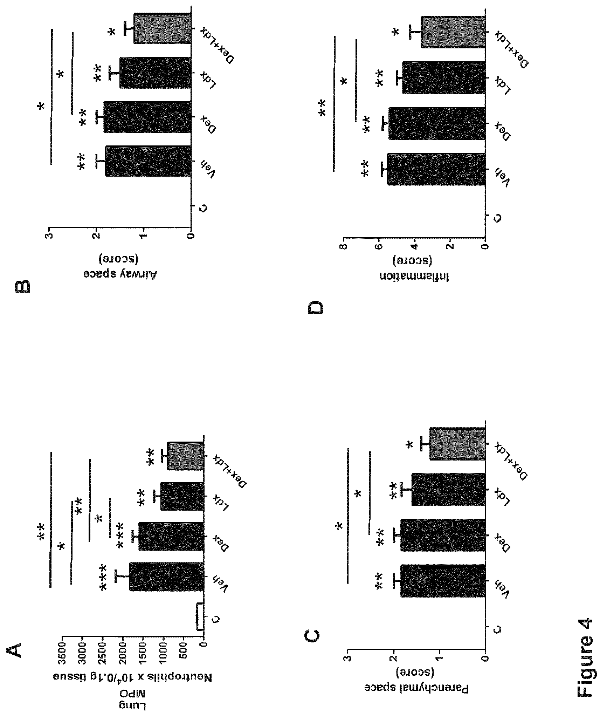

**Figure 4** shows lung MPO activity quantification (panel A) and pulmonary inflammation evaluation in terms of airway space score (panel B), parenchymal space score (Panel C) and inflammation score (panel D), measured as described in Examples 1c) and 1d), from a mouse model of corticosteroid-resistant asthma, after treatment with vehicle (Veh), dexamethasone (Dex), ladarixin (Ldx), or dexamethasone and ladarixin (Dex+Ldx);

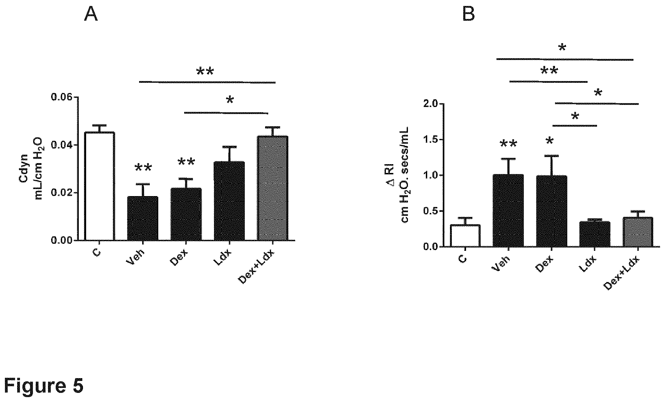

**Figure 5** shows dynamic compliance (Cdyn) and lung resistance evoked by methacholine (ΔRI), measured as described in Example 1e), from a mouse model of corticosteroid-resistant asthma, after treatment with vehicle (Veh), dexamethasone (Dex), ladarixin (Ldx), or dexamethasone and ladarixin (Dex+Ldx).

### DETAILED DESCRIPTION OF THE INVENTION

A first object of the present invention is a combination of a CXCL8 activity inhibitor and a corticosteroid.

Preferably, the above combination is for use in the treatment of corticosteroid-insensitive asthma.

Preferably, said corticosteroid-insensitive asthma is an asthma characterized by a less than 15% improvement in baseline FEV1 (Forced Expiratory Volume at 1 second) after 14 days course of oral prednisolone (40 mg/day) in patients who demonstrate more than 15% improvement in FEV1 following treatment of inhaled β2 agonist.

The term "CXCL8 activity inhibitor" according to the present application refers to any compound able to inhibit, partially or totally, the biological activity of CXCL8. Such a compound can act by decreasing the expression of CXCL8 or of its receptor(s) or by inhibiting the triggering of the intracellular signaling activated by the CXCL8 receptor(s). It is preferred that said CXCL8 activity inhibitor is able to inhibit at least 60%, preferably at least 70%, more preferably at least 80%, even more preferably at least 90% of PMNs chemotaxis induced by an optimal concentration of CXCL8 (1nM) at a concentration equal or below 500 nM, preferably below 100 nM.

According to a preferred embodiment, the CXCL8 activity inhibitor according to the present invention inhibits the activity of CXCL8 mediated by CXCR1 receptor or mediated by both CXCR1 and CXCR2 receptors. Preferably, according to this embodiment, said CXCL8 activity inhibitor is a CXCL8 receptor(s) inhibitor, which inhibits binding of CXCL8 to its receptor(s) or the intracellular signaling activated by the binding of CXCL8 to its receptor(s). Preferably, said CXCL8 receptor inhibitor is either an allosteric inhibitor or an orthosteric antagonist of CXCR1 receptor or of both CXCR1 and CXCR2 receptors.

More preferably, the CXCL8 receptor(s) inhibitor according to the invention has an IC₅₀ value towards CXCR1 receptor in the low nanomolar range, preferably in the range 0.02-5 nanomolar.

According to a preferred embodiment, also in combination with any of the preceding embodiment, said CXCL8 activity inhibitor is selected from small molecular weight molecules, peptides and antibodies, more preferably it is a small molecular weight molecule.

CXCL8 activity inhibitors according to the above definition, able to inhibit the activity of CXCL8 mediated by CXCR1 receptor or mediated by both CXCR1 and CXCR2 receptors, are well known in the art.

To date, several CXCL8 activity inhibitors, such as small molecules, peptides and antibodies, have been disclosed, many of which are currently under undergoing clinical trials or are used in therapy. (Jie Jack, Expert Opinion Ther. Patents, 2001, 11(12), Chao J. et al., Bioorganic & Medicinal Chemistry Letters 17, 2007, p. 3778-3783, Busch-Petersen J. Current Topics in Medicinal Chemistry, 2006, 6, p. 1345-135, Allegretti et al, Immunology Letters 2012, Vol. 145, p. 68-78).

Preferred CXCL8 activity inhibitors according to the present invention are disclosed in WO2000/024710A1 and WO2005/090295 that also disclose their method of synthesis, their activity as CXCL8 receptor inhibitors as well as their use as inhibitors of neutrophils chemotaxis and degranulation induced by CXCL8 and in the treatment of CXCL8 dependent pathologies. These CXCL8 activity inhibitors are CXCL8 receptor(s) inhibitors.

Among these, CXCL8 activity inhibitors particularly preferred are those of the following formula (I): 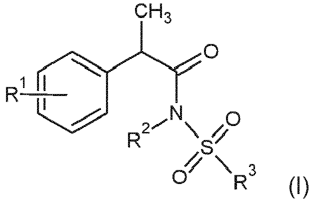 wherein
R¹ is selected from a linear or branched C₁-C₆ alkyl, benzoyl, phenoxy, and trifluoromethanesulfonyloxy;
R² is selected from hydrogen atom and linear or branched C₁-C₃ alkyl; and
R³ is a linear or branched C₁-C₆ alkyl or trifluoromethyl,
and pharmaceutically acceptable salts thereof.

Preferably, R¹ is selected from benzoyl, isobutyl, and trifluoromethanesulfonyloxy. More in particular, R¹ is preferably linked to the phenyl ring in 3- or 4-position. According to the most preferred embodiment, R¹ is 3-benzoyl, 4-isobutyl or 4-trifluoromethanesulfonyloxy. Preferably, R² is selected from hydrogen atom or methyl.

Preferably, R³ is selected from linear or branched C₁-C₆ alkyl, more preferably from linear of branched C₁-C₃ alkyl. According to the most preferred embodiment, R³ is methyl.

Peferably, the chiral carbon of the compounds of formula (I) is in the RS or R configuration, more preferably it is in the R configuration.

The following compounds are particularly preferred among said CXCL8 activity inhibitors of formula (I):
- 2-(4-isobutylphenyl)propionyl methansulfonamide, preferably R-(-)-2-(4-isobutylphenyl)propionyl methansulfonamide (also known as reparixin) and pharmaceutically acceptable salts thereof, preferably the lysine salt thereof, and
- 2-(4-trifluoromethanesulfonyloxy)phenyl]-N-methanesulfonyl propionamide, preferably R(-)-2-(4-trifluoromethanesulfonyloxy)phenyl]-N-methanesulfonyl propionamide (also known as ladarixin or DF2156A) and pharmaceutically acceptable salts thereof, in particular the sodium salt thereof.

Also, other preferred CXCL8 activity inhibitors according to the present invention are those disclosed in WO2010/031835, that also disclose their method of synthesis, their activity as CXCL8 receptor inhibitors as well as their use as inhibitors of neutrophils chemotaxis and degranulation induced by CXCL8 and in the treatment of CXCL8 dependent pathologies. These compounds are CXCL8 receptor(s) inhibitors.

Among these, CXCL8 activity inhibitors particularly preferred are those of the following formula (II): 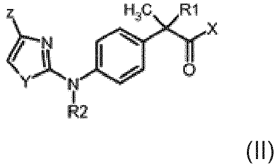 and pharmaceutically acceptable salts thereof, wherein:
R1 is hydrogen;
X is OH;
R2 is hydrogen or linear C1-C4 alkyl,
Y is a heteroatom selected from S, O and N,
Z is selected from linear or branched C1-C4 alkyl, linear or branched C1-C4 alkoxy, halo C1-C3 alkyl and halo C1-C3 alkoxy.

Peferably, the chiral carbon of the compounds of formula (II) is in the is in the RS or S configuration,more preferably in the S configuration.

Particularly preferred among said CXCL8 activity inhibitor of formula (II) is 2-(4-{[4-(trifluoromethyl)-1,3-thiazol-2-yl]amino}phenyl)propanoic acid, preferably (2S)-2-(4-{[4-(trifluoromethyl)-1,3-thiazol-2-yl]amino}phenyl)propanoic acid or the as sodium salt thereof.

The most preferred CXCL8 activity inhibitor is R(-)-2-(4-trifluoromethanesulfonyloxy)phenyl]-N-methanesulfonyl propionamide, preferably as sodium salt.

Any of the CXCL8 activity inhibitors described above may be in a form of a salt with a pharmaceutically acceptable organic or inorganic acid or base or a prodrug or be partially or totally deuterated.

The term "corticosteroid" according to the present application refers to a class of natural steroid hormones and to synthetic analogues thereof that include glucocorticoids and mineralcorticoids.

According to a preferred embodiment of the present invention, the corticosteroids in the combination of the present invention are glucocorticoids. Glucocorticoids are well known in the art.

Preferred glucocorticoids suitable in the combination of the present invention are the glucocorticoids used in the treatment of asthma, preferably amcinonide, beclomethasone dipropionate, betamethasone, betamethasone acetate, betamethasone benzoate, betamethasone di propionate, betamethasone sodium phosphate, betamethasone valerate, budesonide, carbenoxolone sodium, clocortolone acetate, clocortolone pivalate, cloprednol, corticotropin (injection), corticotropin (repository), corticotropin zinc hydroxide, cortisone acetate, cortivazole, descinolone acetonide, dexamethasone, dexamethasone sodium phosphate, diflucortolone, diflucortolone pivalate, flucloronide, flumethasone, flumethasone pivalate, flunisolide, fluocinolone acetonide, fluocinomide, fluocortolone, fluocortolone caproate, fluorometholone, fluperolone acetate, fluprednisolone, fluprednisolone valerate, flurandrenolide, formocortal, fluticasone, hydrocortisone, hydrocortisone acetate, hydrocortisone buteprate, hydrocortisone butyrate, hydrocortisone cypionate, hydrocortisone sodium phosphate, hydrocortisone sodium succinate, hydrocortisone valerate, medrysone, methylprednisolone, methylprednisolone acetate, methylprednisolone sodium phosphate, methylprednisolone sodium succinate, nivazole, paramethasone acetate, prednicarbate, prednisolone, prednisilone acetate, prednisolone hemisuccinate, prednisoline sodium phosphate, prednisolone sodium succinate, prednisiline tebutate, prednisone, prednival, ticabesone propionate, tralonide, triamcinolone, triamcinolone acetonide, triamcinolone acetonide sodium phosphate, triamcinolone diacetate and triamcinolone hexacetonide.

Preferably, in the combination according to the present invention, the glucocorticoids are selected from beclomethasone dipropionate, betamethasone, betamethasone acetate, betamethasone benzoate, betamethasone dipropionate, betamethasone sodium phosphate, betamethasone valerate, budesonide, cortisone acetate, dexamethasone, dexamethasone sodium phosphate, diflucortolone, deflucortolone pivalate, flumethasone, flumethasone pivalate, hydrocortisone, hydrocortisone acetate, hydrocortisone buteprate, hydrocortisone butyrate, hydrocortisone cypionate, hydrocortisone sodium phosphate, fluticasone, hydrocortisone sodium succinate, hydrocortisone valerate, methylprednisilone, methylprednisiline acetate, methylprednisolone sodium phosphate, methylprednisolone sodium succinate, paramethasone acetate, prednisolone, prednisolone acetate, prednisolone hemisuccinate, prednisolone sodium phosphate, prednisolone sodium succinate, prednisolone tebutate, prednisone and triamcinolone.

More preferably, in the combination according to the present invention, the glucocorticoids are selected from beclomethasone, flumethasone, triamcinolone, betamethasone, budesonide, dexamethasone, fluticasone, prednisolone, methylprednisolone, and prednisone. Among these, particularly preferred are dexamethasone, prednisolone, methylprednisolone, and prednisone. The most preferred glucocorticoid according to the invention is dexamethasone.

According to a particularly preferred embiodiment, the combination according to the first object of the invention is a combination of R(-)-2-(4-trifluoromethanesulfonyloxy)phenyl]-N-methanesulfonyl propionamide, preferably as sodium salt (also known as ladarixin or DF2156A), and dexamethasone.

The CXCL8 activity inhibitor and corticosteroid compounds of the present invention may form stable pharmaceutically acceptable acid or base salts with a pharmaceutically acceptable organic or inorganic acid or base, and in such cases administration of a compound as a salt may be appropriate.

Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate.

Examples of base addition salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others. Nontoxic physiologically-acceptable salts are preferred, although other salts may be useful, such as in isolating or purifying the product.

The salts may be formed by conventional means, such as by reacting the free form of the product with one or more equivalents of the appropriate acid or base in a solvent or medium in which the salt is insoluble, such as for example water or ethanol, which is removed under vacuum or by freeze drying.

The present invention also includes the prodrugs, stereoisomers, and enantiomers of the CXCL8 receptor inhibitor and corticosteroid compound described above.

As used herein the term "prodrug" refers to an agent, which is converted into the parent drug in vivo by some physiological chemical process (e.g., a prodrug on being brought to the physiological pH is converted to the desired drug form). Prodrugs are often useful because, in some situations, they may be easier to administer than the parent drug. They may, for instance, be bioavailable by oral administration whereas the parent drug is not. The prodrug may also have improved solubility in pharmacological compositions over the parent drug. An example, without limitation, of a prodrug would be a compound of the present invention wherein it is administered as an ester (the "prodrug") to facilitate transmittal across a cell membrane where water solubility is not beneficial, but then it is metabolically hydrolysed once inside the cell where water solubility is beneficial.

Prodrugs have many useful properties. For example, a prodrug may be more watersoluble than the ultimate drug, thereby facilitating intravenous administration of the drug. A prodrug may also have a higher level of oral bioavailability than the ultimate drug. After administration, the prodrug is enzymatically or chemically cleaved to deliver the ultimate drug in the blood or tissue.

Ester prodrugs of the CXCL8 activity inhibitor and corticosteroid compounds disclosed herein are specifically contemplated. While not intending to be limiting, an ester may be an alkyl ester, an aryl ester, or a heteroaryl ester. The term alkyl has the meaning generally understood by those skilled in the art and refers to linear, branched, or cyclic alkyl moieties. C₁₋₆ alkyl esters are particularly useful, where alkyl part of the ester has from 1 to 6 carbon atoms and includes, but is not limited to, methyl, ethyl, propyl, isopropyl, n-butyl, sec-butyl, iso-butyl, t-butyl, pentyl isomers, hexyl isomers, cyclopropyl, cyclobutyl, cyclopentyl, cyclohexyl, and combinations thereof having from 1 to 6 carbon atoms.

Certain CXCL8 activity inhibitor and corticosteroid compounds may exist in tautomeric forms, and this invention includes all such tautomeric forms of those compounds unless otherwise specified.

Unless otherwise stated, structures depicted herein are also meant to include all stereochemical forms of the structure; i.e., the R and S configurations for each asymmetric centre. Thus, single stereochemical isomers as well as enantiomeric and diastereomeric mixtures of the present compounds are within the scope of the invention. Thus, this invention encompasses each diastereomer or enantiomer substantially free of other isomers (>90%, and preferably >95%, free from other stereoisomers on a molar basis) as well as a mixture of such isomers.

Particular optical isomers can be obtained by resolution of the racemic mixtures according to conventional processes, e.g., by formation of diastereomeric salts, by treatment with an optically active acid or base. Examples of appropriate acids are tartaric, diacetyltartaric, dibenzoyltartaric, ditoluoyltartaric, and camphorsulfonic acid and then separation of the mixture of diastereomers by crystallization followed by liberation of the optically active bases from these salts. A different process for separation of optical isomers involves the use of a chiral chromatography column optimally chosen to maximize the separation of the enantiomers. Still another method involves synthesis of covalent diastereomers by reacting compounds of the invention with an optically pure acid in an activated form or an optically pure isocyanate. The synthesized diastereomers can be separated by conventional means such as chromatography, distillation, crystallization or sublimation, and then hydrolysed to deliver the enantiomerically pure compound. Optically active compounds of the invention can be obtained by using active starting materials. These isomers may be in the form of a free acid, a free base, an ester or a salt.

According to one embodiment, the CXCL8 activity inhibitor and corticosteroid according to the invention are administered simultaneously or sequentially in separate pharmaceutical compositions.

According to an alternative embodiment, the CXCL8 activity inhibitor and corticosteroid are administered together in the same pharmaceutical composition.

Accordingly, a second object of the present invention is a pharmaceutical composition comprising a combination of a CXCL8 activity inhibitor and a corticosteroid according to the first object of the invention, and at least one inert pharmaceutically acceptable excipient.

Preferably, said pharmaceutical composition is for use in the treatment of corticosteroid-insensitive asthma.

Preferably, the pharmaceutical composition of the present invention is prepared in suitable dosage forms comprising an effective amount of a combination of a CXCL8 activity inhibitor and a corticosteroid, a salt thereof with a pharmaceutically acceptable organic or inorganic acid or base, or a prodrug thereof, and at least one inert pharmaceutically acceptable excipient.

The route of administration of the pharmaceutical composition of the present invention is in accord with known methods, e.g., inhalation, injection or infusion by intravenous, parenterally, topically, rectally, nasally, buccally, vaginally or via an implanted reservoir, or by sustained release systems. The term parenteral as used herein includes intravenous, intraperitoneal, intracerebral, intrathecal, intracranial, intramuscular, intraarticular, intrasynovial, intrasternal, intraocular, intraarterial, subcutaneous, intracutaneous or intralesional injection or infusion techniques.

In one preferred embodiment, the invention provides for the treatment of corticosteroid-insensitive asthma by direct administration of the pharmaceutical composition of the present invention to the respiratory tract. The pharmaceutical composition of the present invention can be used in a wide variety of devices that are designed for the direct delivery of pharmaceutical compositions and therapeutic formulations to the respiratory tract. In one aspect of the present invention, the pharmaceutical composition of the present invention is administered in aerosolized or inhaled form. The pharmaceutical composition of the present invention can be administered in an aerosol formulation as a dry powder or in a solution or suspension with a diluent.

In the present description and in the following claims, the wording "effective amount" means an amount of a compound or composition which is sufficient enough to significantly and positively modify the symptoms and/or conditions to be treated (e.g., provide a positive clinical response). The effective amount of an active ingredient for use in a pharmaceutical composition will vary with the particular condition being treated, the severity of the condition, the duration of the treatment, the nature of concurrent therapy, the particular active ingredient(s) being employed, the particular pharmaceutically-acceptable excipient(s)/carrier(s) utilized, and like factors within the knowledge and expertise of the attending physician.

As described herein, the pharmaceutical composition of the present invention comprises a combination of a CXCL8 activity inhibitor and a corticosteroid together with a pharmaceutically acceptable excipient, which, as used herein, includes any and all solvents, diluents, or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions.

Moreover, the composition of the present invention may be formulated into inhalable or injectable dosage forms such as solutions, suspensions, and emulsions by further adding diluents, dispersants, and surfactants.

Further, the composition of the present invention may be suitably formulated using appropriate methods known in the art or by the method disclosed in Remington's Pharmaceutical Science (recent edition), Mack Publishing Company, Easton Pa.

The terms "pharmaceutically acceptable" and "physiologically acceptable" are intended to define, without any particular limitation, any material suitable for preparing a pharmaceutical composition to be administered to a living being.

The dosage forms can also contain other traditional ingredients such as: preservatives, stabilizers, surfactants, buffers, salts for regulating osmotic pressure, emulsifiers, sweeteners, colorants, flavourings and the like.

The amount of said CXCL8 activity inhibitor and corticosteroid compounds in the pharmaceutical composition of the present invention is the the amount usually employed when these are used individually.

The amount can vary over a wide range depending on known factors, for example, the molecule used, the severity of the disease, the patient's body weight, the dosage form, the chosen route of administration, and the number of administrations per day. However, a person skilled in the art can determine the optimum amount in easily and routinely manner.

Also specific dosage and treatment regimens for any particular patient will depend upon a variety of factors, including for example the activity and half life of the specific compound employed, the age, body weight, general health status, sex, diet, severity and course of the disease.

The dosage forms of the pharmaceutical composition of the present invention can be prepared by techniques that are familiar to a pharmaceutical chemist, and comprise mixing, granulation, compression, dissolution, sterilization and the like.

A third aspect of the present invention relates to a method of treating corticosteroid-insensitive asthma in a subject in need thereof, comprising administering to the subject a pharmaceutical composition comprising a combination of an CXCL8 activity inhibitor and a corticosteroid according to the second aspect of the invention.

The invention will be further described in the following examples, which do not limit the scope of the invention described in the claims.

### EXPERIMENTAL SECTION

In the Figures, all results are expressed as the mean ± SEM. Normalized data were analyzed by One-Way ANOVA with Tukey post-test, using the software GraphPad Prism 7.0. Differences were considered significant at P< 0.05. n=8; * P<0.05; ** P< 0.01; *** p< 0.001.

### Example 1

The investigation was conducted by using a Th17 glucocorticoid-insensitive asthma mouse model previously described (Campa CC et al., Nat Commun (2018), v. 9, p. 5232 and Dejager Let al., Mucosal Immunol (2015), v. 8, p 1212-25).

In details, BALB/c mice received systemic immunization by subcutaneous injection of 20 µg of chicken egg ovalbumin (OVA grade V, >98% pure; Sigma, St Louis, MO) in saline, emulsified in 75 µL CFA (Sigma-Aldrich) at day 0.

From day 28 after first immunization to day 31, mice were treated with PBS (vehicle), ladarixin, dexamethasone or ladarixin/dexamethasone as described below, and 60 minutes later exposed to aerosolized ovalbumin (1% in saline) for 20 minutes per day. Ladarixin was administered orally by gavage (10 mg/Kg in saline 0.9%), as previously described (Bertini et al., Br J Pharmacol (2012), v.165, p. 436-54), once a day. Dexamethasone was administered subcutaneously (5 mg/Kg in saline), once a day.

### a) Lekocytes leakage in airways

An assessment of total leukocytes content in airways was carried out by performing bronchoalveolar lavage fluid (BALF) as previously described (Russo et al Am J Respir Cell Mol Biol (2009), v. 40, p. 410-21; Campa CC et al., Nat Commun (2018), v. 9, p. 5232). Briefly, an incision was made in the trachea where a 1.7mm outside diameter polyethylene catheter was inserted. Then, the airways were washed 2 times with 1mL of phosphate buffer saline (PBS). The samples were centrifuged at 300 x g for 10 minutes at 4 °C, the pellet was collected, resuspended in 100 µL of 3% BSA in PBS and a sample from this solution was used to assess the total leukocytes in the airways. The remaining was centrifuged at 300 x g for 10 minutes at 4 °C on a histology glass slide to assess differential counting of macrophages, lymphocytes, eosinophils and neutrophils using an optical microscopy. Total protein was quantified in the BALF supernatant using a Bio-Rad protein assay Dye Reagent (Bio-Rad, USA) to measure possible protein leakage as previously described (Campa CC et al., Nat Commun (2018), v. 9, p. 5232).
The results are illustrated in Figure 1.

As can be seen from the data, significant leukocyte influx into the airways with predominance of neutrophils and macrophages, and less extended numbers of eosinophils and lymphocytes, with increased protein leakage into airways is observed in the vehicle treated mice.
Treatment with ladarixin reduces total leukocyte influx into alveolar space, by marked reduction of macrophages and neutrophils, while dexamethasone fails to reduce the recruitment of these cells. However, surprisingly, the combined treatment of ladarixin and dexamethasone produces a synergistic effect with a significantly greater decrease in total leukocyte influx and number of infiltrated macrophages and neutrophils compared to ladarixin alone.
A synergistic effect is also observed on eosinophil recruitment into the airways, which is slightly reduced by treatment with dexamethasone and increased by ladarixin administration. Surprisingly, co-treatment with dexamethasone and ladarixin produces a reduction in the recruitment of these cells greater than that observed with dexamethasone alone.

### b) Cytokines quantification in lung tissue

The levels of IL17A and TNF-α were also measured in the different experimental mice groups. Briefly, after BALF, right lung was removed and a sample of 100 mg was homogenized in 1 mL of PBS containing anti-proteases (0.1 mM phenylmethylsulfonyl fluoride, 0.1 mM benzetonium chloride, 10 mM EDTA and 20 Kl aprotinin (A). The homogenate was centrifuged 8,000 x g for 10 minutes at 4 °C. The supernatant was frozen for further ELISA assay. Levels of IL-17A and TNF-α were measured by ELISA using commercial DuoSet kits from R&D Systems, according to the instructions of the manufacturer. Results, shown in Figure 2, are expressed as pg of cytokines per ml of lung lysate (pg/ml).

As shown in Figure 2, the Th17 cytokines IL-17A and TNF-α were significantly decreased by treatment with ladarixin alone or ladarixin in combination with dexamethasone.

### c) Neutrophil peroxidase activity (MPO)

MPO assay was performed on the lung tissue in the different experimental mice groups.

In details, MPO assay was performed in a 96-well microplate by adding, in each well, 25 µL of 3,3'-5,5'-tetramethylbenzidine (TMB Sigma) diluted in dimethyl sulfoxide (DMSO; Merck, Darmstadt, Germany) at a final concentration of 1,6 mM; 100 µL of H₂O₂ 0.02% vol/vol in PBS (pH 5.4) containing HTAB and 25 µL of processed tissue supernatant. The reaction started, at 37 °C, by adding the TMB solution and the supernatant to the microplate and, after 5 min, H₂O₂ was added followed by a new incubation at 37 °C for 5 min. The reaction was stopped by adding 100 µL of H₂SO₄ 1M and quantified at 450 nm wavelengths in a spectrophotometer (VERSA max, Molecular Devices, CA).

Semiquantitative histopathology analyses were performed as described (Campa CC et al., Nat Commun (2018), v. 9, p. 5232). Briefly, ten random images per lung were acquired at 200x and pulmonary inflammation score was recorded according to a six grade scale where "0" corresponded to less than 1% of lung tissue area inflamed, "1" where up to 9% of lung area was affected by inflammation, "2" when 10% to 29% of lung tissue area was affected, "3" if 30% to 49% of lung tissue area was affected, "4" if 50% to 69% of lung tissue area was affected and "5" if more than 70% of the lung tissue area was affected by inflammation.

As shown in Figure 4, when the leukocyte infiltration into lung tissue was assessed by MPO, a synergistic effect between dexamethasone and ladarixin treatments was observed. Ladarixin, but not dexamethasone alone, is able to prevent the neutrophil accumulation in lung parenchyma after OVA exposure. However, the addition of dexamethasone to ladarixin treatment further decreases neutrophils infiltration.

### d) Histological analysis

After BALF, left lung was removed and fixed in formalin 4% in PBS (pH 7.4), as previously described. The tissue was dehydrated gradually in ethanol, embedded in paraffin, and cut into 4µm sections and stained with H&E, periodic acid Schiff (PAS) or Gomori's trichrome³⁶. Photomicrographs were obtained at 400x magnification and were analyzed using the software Image Pro Plus (Image-Pro Plus 4.1, Media Cybernetics, USA) under light microscopy. Nine to twelve bronchial areas per lung had their PAS or Gomori's trichrome content quantified and the results were expressed as positive area (pixels). Fibrosis was determined using Gomori's trichrome stained slices. Semiquantitative histopathology analyses were performed as described by Campa CC et al., Nat Commun (2018), v. 9, p. 5232 *et al,* 2018. Briefly, ten random images per lung were acquired at 200x and pulmonary inflammation score was recorded according to a six grade scale, where "0" corresponded to less than 1% of lung tissue area inflamed, "1" where up to 9% of lung area was affected by inflammation, "2" when 10% to 29% of lung tissue area was affected, "3" if 30% to 49% of lung tissue area was affected, "4" if 50% to 69% of lung tissue area was affected, "5" if more than 70% of the lung tissue area was affected by inflammation.

By evaluating the lung histology and pathology score, we could observe that there is no inflammation in control mice, but in Vehicle mice inflammation score is very high. Treatment with ladarixin but not dexamethasone is effective in reducing inflammation (see Figure 4, panel D) but a further decrease in inflammatory scored is observed when dexamethasone si associated to ladarixin, evidencing a synergistic effect when the two compounds are associated. These data are also confirmed by the histological images in Figure 3, wherein arrows indicate peribronchial inflammation.

### e) Assessment of pulmonary mechanics

Mice form different experimental groups were anesthetized with a subcutaneous injection of ketamine and xylazine (130 mg/Kg ketamine and 8.5 mg/Kg xylazine) to maintain spontaneous breathing under anesthesia. Mice were tracheostomized and placed in a whole-body plethysmograph, connected to a computer-controlled ventilator (Forced Pulmonary Maneuver System®, Buxco Research Systems®, Wilmington, North Carolina USA). Under mechanical respiration the Dynamic Compliance (Cdyn) and Lung Resistance (RI) were determined by Resistance and Compliance RC test.

The anesthetized mice were instrumented to directly measure respiratory flow and airway pressure. From these direct measurements of airflow (RI) and pressure (Cdyn) are computed .

The results obtained, shown in Figure 5 Th17 airway inflammation leads to tissue dysfunction such as reduced airway flow and pulmonary elasticity which is refractory to dexamethasone treatment. However, the co-treatment with ladarixin plus dexamethasone preserved Dynamic Compliance (Figure 5, panel A). Most importantly, ladarixin or dexamethasone plus ladarixin were able to reduce airway hyperreactivity evoked by methacholine (Figure 5, panel B).

Collectively, these results demonstrated in corticosteroid-insensitive Th17 neutrophilic airway inflammation and hyperreactivity induced by OVA nebulization in mice ladarixin is able to restore the anti-inflammatory effect of dexamethasone via a synergistic mechanism.

Indeed, CXCR1 and CXCR2 antagonism through oral administration of ladarixin plus dexamethasone prevented neutrophilic airway inflammation and protein leakage, tissue remodeling, bronchial hyperreactivity and improved pulmonary mechanics in GC-insensitive asthma model.

## Claims

1. A combination of (i) an CXCL8 activity inhibitor, a salt thereof with a pharmaceutically acceptable organic or inorganic acid or base, or a prodrug thereof, and (ii) a corticosteroid, a salt thereof with a pharmaceutically acceptable organic or inorganic acid or base, or a prodrug thereof.

2. The combination as claimed in claim 1, wherein said CXCL8 activity inhibitor inhibits the activity of CXCL8 mediated by CXCR1 receptor or mediated by both CXCR1 and CXCR2 receptors and/or it is is a CXCL8 receptor inhibitor.

3. The combination as claimed in claim 1 or 2, wherein said CXCL8 activity inhibitor is a small molecule, an antibody or a peptide.

4. The combination as claimed in claim 1 to 3, wherein said CXCL8 activity inhibitor is a compound of formula (I): 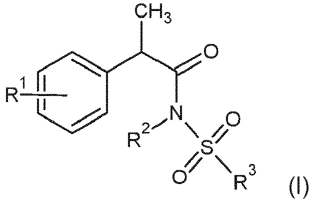 wherein
R¹ is selected from linear or branched C₁-C₆ alkyl, benzoyl, phenoxy, and trifluoromethanesulfonyloxy;
R² is selected from hydrogen atom and linear or branched C₁-C₃ alkyl; and
R³ is a linear or branched C₁-C₆ alkyl or trifluoromethyl,
or a pharmaceutically acceptable salt thereof.

5. The combination as claimed in claim 1 to 4, wherein said CXCL8 activity inhibitor is selected from:
- 2-(4-isobutylphenyl)propionyl methansulfonamide, preferably R-(-)-2-(4-isobutylphenyl)propionyl methansulfonamide (also known as reparixin) and pharmaceutically acceptable salts thereof, preferably the lysine salt thereof, and
- 2-(4-trifluoromethanesulfonyloxy)phenyl]-N-methanesulfonyl propionamide, preferably R(-)-2-(4-trifluoromethanesulfonyloxy)phenyl]-N-methanesulfonyl propionamide (also known as ladarixin or DF2156A) and pharmaceutically acceptable salts thereof, in particular the sodium salt thereof.

6. The combination as claimed in claim 1 to 3, wherein said CXCL8 activity inhibitor is a compound of formula (II): 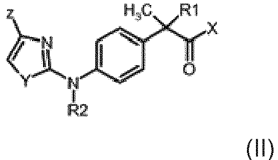 wherein:
R1 is hydrogen;
X is OH;
R2 is hydrogen or linear C1-C4 alkyl,
Y is a heteroatom selected from S, O and N,
Z is selected from linear or branched C1-C4 alkyl, linear or branched C1-C4 alkoxy, halo C1-C3 alkyl and halo C1-C3 alkoxy,
or a pharmaceutically acceptable salt thereof.

7. The combination as claimed in claim 6, wherein said CXCL8 activity inhibitor is 2-(4-{[4-(trifluoromethyl)-1,3-thiazol-2-yl]amino}phenyl)propanoic acid, preferably (2S)-2-(4-{[4-(trifluoromethyl)-1,3-thiazol-2-yl] amino} phenyl) propanoic acid or the sodium salt thereof.

8. The combination as claimed in claim 1 to 7, wherein said corticosteroid is a glucocorticoid selected from amcinonide, beclomethasone dipropionate, betamethasone, betamethasone acetate, betamethasone benzoate, betamethasone di propionate, betamethasone sodium phosphate, betamethasone valerate, budesonide, carbenoxolone sodium, clocortolone acetate, clocortolone pivalate, cloprednol, corticotropin (injection), corticotropin (repository), corticotropin zinc hydroxide, cortisone acetate, cortivazole, descinolone acetonide, dexamethasone, dexamethasone sodium phosphate, diflucortolone, diflucortolone pivalate, flucloronide, flumethasone, flumethasone pivalate, flunisolide, fluocinolone acetonide, fluocinomide, fluocortolone, fluocortolone caproate, fluorometholone, fluperolone acetate, fluprednisolone, fluprednisolone valerate, flurandrenolide, formocortal, fluticasone, hydrocortisone, hydrocortisone acetate, hydrocortisone buteprate, hydrocortisone butyrate, hydrocortisone cypionate, hydrocortisone sodium phosphate, hydrocortisone sodium succinate, hydrocortisone valerate, medrysone, methylprednisolone, methylprednisolone acetate, methylprednisolone sodium phosphate, methylprednisolone sodium succinate, nivazole, paramethasone acetate, prednicarbate, prednisolone, prednisilone acetate, prednisolone hemisuccinate, prednisoline sodium phosphate, prednisolone sodium succinate, prednisiline tebutate, prednisone, prednival, ticabesone propionate, tralonide, triamcinolone, triamcinolone acetonide, triamcinolone acetonide sodium phosphate, triamcinolone diacetate and triamcinolone hexacetonide.

9. The combination as claimed in claim 1 to 8, wherein said glucocorticoid is selected from dexamethasone, prednisolone, methylprednisolone, and prednisone, a salt thereof with a pharmaceutically acceptable organic or inorganic acid or base, or a prodrug thereof.

10. The combination as claimed in claims 1 to 9, wherein said CXCL8 activity inhibitor is R(-)-2-(4-trifluoromethanesulfonyloxy)phenyl]-N-methanesulfonyl propionamide, preferably as sodium salt, and said corticosteroid is dexamethasone.

11. A pharmaceutical composition comprising a combination as claimed in claims 1 to 10, and at least one inert pharmaceutically acceptable excipient.

12. A combination as claimed in claims 1 to 10 or a pharmaceutical composition as claimed in claim 11 for use in the treatment of corticosteroid-insensitive asthma.

13. A composition for use as claimed in claim 12, wherein said asthma is an asthma **characterized by** a less than 15% improvement in baseline FEV1 (Forced Expiratory Volume at 1 second) after 14 days course of oral prednisolone (40 mg/day) in patients who demonstrate more than 15% improvement in FEV1 following treatment of inhaled β2 agonist
